**Europäisches Patentamt**

**European Patent Office**

**Office européen des brevets**

(19)

(11) **EP 1 564 780 A2**

(12) **EUROPEAN PATENT APPLICATION**

| | |
|---|---|
| (43) Date of publication:<br>**17.08.2005 Bulletin 2005/33** | (51) Int Cl.[7]: **H01J 27/26**, H01J 49/16,<br>H01J 49/04 |

(21) Application number: **04257354.3**

(22) Date of filing: **26.11.2004**

| | |
|---|---|
| (84) Designated Contracting States:<br>**AT BE BG CH CY CZ DE DK EE ES FI FR GB GR**<br>**HU IE IS IT LI LU MC NL PL PT RO SE SI SK TR**<br>Designated Extension States:<br>**AL HR LT LV MK YU** | (72) Inventors:<br>• **Nelson Alexander IV, James**<br>  **Lansdale, Pennsylvania 19446 (US)**<br>• **Saucy, Daniel Alain**<br>  **Harleysville, Pennsylvania 19438 (US)** |
| (30) Priority: **08.12.2003 US 527897** | (74) Representative: **Buckley, Guy Julian et al**<br>**ROHM AND HAAS (UK) LTD.**<br>**European Patent Department**<br>**28th Floor, City Point**<br>**One Ropemaker Street**<br>**London EC2Y 9HS (GB)** |
| (71) Applicant: **Rohm and Haas Company**<br>**Philadelphia, Pennsylvania 19106-2399 (US)** | |

(54) **Apparatus for determining molecular weight of polymers**

(57)    The invention is directed to an apparatus and a method for direct mass analysis of molecules and macromolecules, including polymers, using charge reduced gas phase analyte ions generated from analyte electrosprays coupled to a time of flight mass spectrometer (TOF-MS). Molecules and macromolecules, including polymers, analyzed in accordance with the invention include both water soluble and water insoluble polymers having weight average molecular weights between 1 kilodaltons (kD) and 500,000 kD.

**EP 1 564 780 A2**

**Description**

The present invention relates to an apparatus and a direct method for determining molecular weight of molecules and macromolecules. More particularly, the invention is directed to the direct mass analysis of polymers using charge reduced polymer ions generated from polymer electrosprays coupled to a time of flight mass spectrometer (TOF-MS).

Determination of the molecular weight distribution of polymers is an important aspect of polymer characterization. The conventional method for determining the molecular weight distribution (MWD) of a polymer is the use of Gel Permeation Chromatography (GPC) relative to some polymer standard whose MWD has been accurately determined. However, the molecular weight distribution for many polymers cannot be determined using GPC. For a successful determination of MWD using GPC, the polymer must dissolve in a solvent that is compatible with GPC column packing materials and avoids adsorption interactions with the column. Mass spectrometry (MS) is another method to determine the MWD of a polymer. However, while mass spectrometric detection provides an effective means for identifying a wide variety of molecules, its use for analyzing high molecular weight compounds is currently hindered by problems related to producing gas phase ions attributable to a given analyte. In particular, the application of mass spectrometric analysis to determine the composition of mixtures of important biological compounds and industrial polymers is severely limited by experimental difficulties related to low sample volatility and unavoidable fragmentation during vaporization and ionization processes. As a result of these limitations, the potential for quantitative analysis of samples containing biological macromolecules and polymers via mass spectrometry remains largely unrealized. In addition, polymer ions are often produced having a range of charge states (z). When this range is convoluted with the broad MWD of the polymer to be analyzed, the result is severe spectral overlap, since the ions are separated according to their m/z ratio. Such overlap often makes it impossible to determine an accurate MWD of the polymer once the average molecular weight exceeds approximately 10,000 daltons.

Typical limitations associated with currently available systems and methods for mass spectral analysis are those described in U.S. Patent No. 6,649,907 B2, which discloses a charge reduction ionization ion source for use in mass spectral analysis of biological compounds such as peptides, nucleotides, glycoproteins, proteins, DNA and lipids whose measured mass range is up to 18000 amu. Unfortunately, attempts to perform the mass analysis on a mixture of water soluble polymers, a mixture of polyethylene glycols (PEG) having average molecular weights of 2,000 daltons and 10,000 daltons respectively, show how poor the mass resolution is (peak broadening) as a result of multiple charging and the corona discharge ionization source. Moreover, the spectral overlap problem for biological molecules is practically nonexistent and multiple charging, rather than being a hindrance, is often used to advantage for particles whose mass is beyond the range of a mass analyzer, but whose m/z, due to multiple charging, is not. In addition to water soluble polymers, many industrially important and commercially available polymers are not water soluble and it would be desirable to have a practical and effective method for preparing electrosprays of such polymers and an apparatus that couples a electrospray generator effective at producing stable polymer electrosprays with a time of flight mass sprectrometer (TOF-MS) to directly perform mass analysis of such polymers. One advantage to the method is that molecular weight axis of measured polymer data is absolute. It is desirable, therefore, to provide a direct method of determining molecular weight distribution of polymers at high resolution for polymer particle sizes ranging from 1 to 100 nanometers (nm). It is also desirable to generate electrospray polymer clusters including one or more polymer molecules clustered in each electrospray droplet (unimers n = 1, dimers n = 2, hexamers n = 6 and higher clusters). Such an apparatus and method would provide an unprecedented direct mass analysis of industrial polymers whose molecular weights span a range from several thousand daltons (corresponding to polymer particle sizes of 1-3 nm) to several hundred million daltons (corresponding to polymer particle sizes of 15-100 nm); polymers whose molecular weight distribution cannot be performed using conventional methods including GPC and conventional electrospray ionization mass spectrometery (ESI-MS).

Accordingly, the invention provides an apparatus for direct mass analysis of macromolecules having molecular weights beyond mass ranges of conventional mass spectrometers. Inventors have discovered an apparatus and method for direct mass analysis of polymers by generating charge reduced electrosprays of both water soluble polymers and water insoluble polymers using solvents having high dielectric constants. Polymer ions formed in an electrospray carry a level of charge proportional to their length and hence their mass. The number of charges on the resulting polymer ions is reduced to a value approaching unity using a radioactive source or a corona discharge source. The polymer electrosprays generated are fed in to a time of flight mass spectrometer (TOF-MS) for direct mass analysis. The inventors also discovered that clusters of polymer ions in each electrospray droplet including from 1 up to n molecules of the polymer are generated by varying the polymer concentration in the electrospray droplet.

The invention provides an apparatus for the direct mass analysis of molecules and macromolecules comprising: an electrospray generator for producing electrosprays of an analyte comprising molecules and macromolecules, the electrospray generator including an ionization source for charge reduction of gas phase analyte ions coupled with a time of flight mass spectrometer for direct mass analysis of the analyte.

The invention also provides a direct method for determining molecular weight distribution of molecules and macromolecules comprising the steps of:

(a) generating an electrospray of an analyte comprising molecules and macromolecules; and
(b) measuring the mass ratio of the analyte by directing the charge reduced electrospray of the analyte into a time of flight mass spectrometer.

As used herein, a dalton is a unit of atomic mass equal to 1/12 the most abundant isotope of carbon ($^{12}$C), which has an atomic mass of 12; 1 dalton = 1 atomic mass unit (amu). Polymers analyzed in accordance with the invention include both water soluble and water insoluble polymers having weight average molecular weights between 1 kilodalton (kD) and 500,000 kD. As used herein, the term "water soluble", as applied to polymers, indicates that the polymer has a solubility of at least 1 gram per 100 grams of water, preferably at least 10 grams per 100 grams of water and more preferably at least about 50 grams per 100 grams of water. The term "water insoluble", as applied to polymers, refers to polymers which have low or very low water solubility of less than 1 gram per 100 grams of water. The term macromolecules refers to large molecules, including polymers, having a molecular weight of greater than 10,000 daltons.

As used herein, the term "cluster ions" refers to a series of internally dependent masses generated from a primary reference polymer mass standard that includes from 1 (unimer) to n molecules (e.g. n = 6 refers to a hexamer) of the primary reference polymer mass standard. The number of cluster ions varies with the concentration of the polymer in a droplet. The advantage of generating cluster ions is that it extends the mass range a factor n(m). For example, a 50kD polyethylene glycol (PEG) unimer has an equivalent measured mobility, mass and density with a 12kD PEG tetramer (or 4 X 12kD = 48 kD, n = 4).

According to the invention, electrosprays of water soluble polymers and water insoluble polymers are produced using an electrospray source (ES), also referred to as an electrospray apparatus (U. S. Pat. No. 5,873,523) and an electrospray droplet generator (U.S. Patent Application Publication No. 2003/0136680 A1). Any conventional or commercially available electrospray source or generator is usefully employed in accordance with the invention. The electrospray apparatus used in the invention is similar in design to an apparatus described by Kaufman *et al.* in *Analytical Chem.,* Vol. 68, pp. 1895-1904 (1996). A similar electrospray droplet generator is commercially available as TSI Model 3480 Electrospray Aerosol Generator (TSI Inc., St. Paul, Minnesota). An alternative electrospray apparatus is described in U. S. Patent No. 5,873,523.

The ES produces uniform, nanometer sized electrosprays (also referred to as droplets and aerosols) from electrically conducting solutions including water soluble polymers and water insoluble polymers as analytes. The term electrospray refers to droplets (and aerosols) having a well defined size distribution that are generated by feeding a liquid with sufficient electrical conductivity through a capillary while maintaining an electrical potential of several kilovolts (kV) relative to a reference electrode positioned at a specified distance away from the capillary (centimeters, cm to millimeters, mm). The term aerosol refers to a nanometer (nm) sized solution, including dispersion and suspension, of an analyte suspended in air in the form of droplets. The electrosprays of the present invention are produced under a large applied electrostatic potential from the ES as solvent droplets having a well defined size distribution containing a dissolved polymer. The term analyte refers to a solid analyte (polymer) dissolved in a solvent that yields a solution. The term dissolved polymer and polymer solution is understood in the context of the present to include suspensions and dispersions of polymers, typically fine suspensions and dispersions of polymers. Electrosprays of fine dispersions and suspensions of analyte are produced using a solvent or liquid carrier. The suspensions and dispersions comprise homogeneous or heterogeneous mixtures of the analyte in the solvent or liquid carrier.

The ES of the invention includes a capillary having an exit for ejecting a liquid that is charged to a high electric potential by a high voltage power supply. A typical example of a capillary is a silica capillary. Alternative capillaries are made from conductive materials. A reference electrode is positioned a specified distance away (cm) from the capillary. A gas source is used to establish a region of gas immediately in the region of the capillary exit. A typical gas source includes for example air and carbon dioxide. The potential difference between the capillary exit and the electrode in the ES is sufficient to both establish electrosprays containing highly uniform sized droplets.

The droplets including polymer analyte are carried by a laminar gas flow where they rapidly de-solvate and dry, forming neutral and charged polymer particles that are exposed to an ionization source (*e.g.* alpha emitting radiation source), which reduces the charge of the particles to values approaching unity as the droplets evaporate. The level of exposure to the alpha radioactive source (*e.g.* typically 5 milliCuries $^{210}$Po) is controlled by covering its active region with metal foil. It is regulated so that polymer analytes cease to display a peak associated to a multiply charged species. The droplets are allowed ample time to evaporate before the dry polymer particles are charged reduced.

The polymer particles carry the same amount of charge as did the droplets that were initially electrosprayed. The alpha-emitter source charge reduction process produces reliable, well characterized streams of charged polymer particles. As used herein, well-characterized means that, although the fraction of singly charged polymer particles depends on particle diameter, the relationship between particle diameter and the fraction of polymer particles carrying a single charge is well established. Alternative methods are used to ensure that an entering stream of charged polymer particles exits with particles having no more than a single charge, including an alternating current corona that produces secondary electrons having the same charge state reduction as the alpha radiation source.

According to one embodiment of the method, electrosprays are generated having a particle size distribution such that they include only one polymer molecule as the analyte without the need to use significantly low polymer analyte concentration. In the case of water soluble polymers (e.g. PEG), water is used as a solvent that also include small amounts of one or more electrolytes (*e.g.* 10mM ammonium acetate). In the case of water insoluble polymers (*e.g.* polystyrene, PS), solvents having a high dielectric constant are used (e.g. N-methyl-2-pyrrolidinone, NMP) that also include small amounts of one or more electrolytes (*e.g.* 10 mM trifluoroacetic acid, TFA). For a given droplet diameter and polymer molecular weight, the corresponding polymer concentration that provides one polymer chain per droplet is calculated. For example a 100kD PEG having an initial droplet diameter of 50 nm provides a single PEG chain at a concentration of 2538 ppm. The same polymer having initial droplet diameters of 100 nm, 500 nm, 2 um and 10 um provides a single PEG chain at a concentrations of 317 ppm, 2.5 ppm, 0.04 ppm and 0.0003, respectively. Thus to maximize signal, the droplet diameter is minimized and electrospraying generates droplets having narrow size distributions. Increasing the concentration of polymer in the droplet provides multiple polymer molecules (chains) provides clusters of molecules in the same dry polymer particles, referred to as cluster ions. The number of clusters ions, n formed, is used a reasonable approximation of a single molecule (chain) polymer of higher molecular weight m, thereby extending the molecular weight range by a factor of n(m). For example, a hexamer of a 120kD PEG is the equivalent of a single chain (molecule) 720kD PEG (6 X 120 kD = 720 kD).

The ES apparatus delivers charge reduced polymer particles to a mass analyzer to determine the mass to charge ratios (m/z) of the gas phase polymer ions generated from the polymer electrospray. According to an exemplary embodiment, the mass analyzer is a time of flight mass spectrometer (TOF-MS) equipped with a detector system suitable for providing accurate measurements of m/z ratios for compounds having molecular masses greater than 10,000 daltons. Mass analysis of molecules and macromolecules, including polymers, having weight average molecular weights between 1 kilodaltons (kD) and 500,000 kD is direct using the apparatus and it does not suffer the inherent limitations in conventional ESI-MS and matrix assisted laser desorption ionization mass spectrometry (MALDI-MS), namely spectral congestion from multiple masses, each in a large number of different charge states. The latter two devices have been unsatisfactory for determining the mass distributions of industrial polymers, while the device of the invention provides accurate, direct mass analysis, including mass distribution, of industrial polymers.

The apparatus of the invention also provides a direct method for determining molecular weight distribution of molecules and macromolecules comprising the steps of:

> (a) generating an electrospray of an analyte comprising molecules and macromolecules; and
> (b) measuring the mass ratio of the analyte by directing the charge reduced electrospray of the analyte into a time of flight mass spectrometer.

According to separate exemplary embodiments, alternative mass analyzers include but are not limited to, for example, differential mobility analyzers (DMA), quadrupole mass spectrometers, tandem mass spectrometers, ion traps and combinations of the mass analyzers.

According to the DMA embodiment, the ES apparatus delivers charge reduced polymer particles to a differential mobility analyzer (DMA), where the particle size of the charged polymer particles is determined. Ion electrical mobility is a physical property of an ion and is related to the velocity an ion acquires when it is subjected to an electrical field. Electrical mobility, Z, is defined from the mathematical relationship Z = V/E, where V is the terminal velocity and E is the electrical field. Particle diameter is determined from the relationship $Z = neC_c/3\pi\eta d$ by substituting the following terms, where n is the number of charges on the particle ($n = 1$), $e = 1.6 \times 10^{-19}$ Coulombs/charge, $C_c$ is the particle size dependent slip correction factor, $\eta$ is the gas viscosity and d is the particle diameter. Solving for particle diameter leads to the relationship $d = [neC_c/3\pi\eta][E/V]$ provides an explicit relationship for particle diameter as function of known parameters. By varying E, different particle diameters of the charged polymer particles are obtained. The present invention provides a method for measuring polymer ion electrical mobility using a high resolution DMA and developing an accurate calibration method for relating measured polymer ion mobility to polymer mass.

The DMA combines a flow field and an electric field to separate ions in space rather than time. The DMA used in the invention was described by T. Eichler in a thesis presented to Fachhochschule Offenburg, Germany (May 1997) entitled, "Differential Mobility Analyzer for Ions and Nanoparticles: Laminar Flow at High Reynolds Numbers" with improvements of a short Vienna-type DMA with a large laminarizing inlet described by Herrmann *et al.* at the annual meeting of AAAR in St. Louis, MS, October 6-10, 2000. In practice, a typical DMA cannot measure the size distribution of particles smaller than 2 nm. However, they are easily used in the invention to measuring polymers having Mw >10 kD. High resolution DMA have been designed and built that can measure size distribution of particles below 1 nm. For such high resolution DMA as described in U. S. Pat. Appl. No. 2003/0116708 A1, laminar flow is unconventionally high and corresponds to Reynolds numbers (Re) from 10,000 to 30,000 and greater, as calculated from the mean velocity in the analyzing section and the separation between cylindrical electrodes (0.5 centimeters), while maintaining laminar flow. The term "Reynolds number" is a dimensionless number which is equal to the product of the density of a fluid, the velocity of the fluid and a characteristic length, all divided by the viscosity coefficient of the fluid. While such high Re numbers are required to measure ion mobilities for organic molecules having small Mw, it is not required to accurately measure ion mobilities for polymers having Mw greater than 1kD. One advantage is the method for accurately determining polymer masses requires operating the DMA at Reynolds numbers less than 2000 (Re < 2000) while maintaining laminar flow.

Charge reduced polymer ions are injected through the DMA, which separates the charged polymer ions according to their diameter and mobility. The detector sensing the mobility selected polymer particles is an electrometer commercially supplied by Lazcano Inc. having a response time of about 1 second and a noise level of about $10^{-16}$ Amperes. DMA's with mobility resolutions of better than 2.7% full width at maximum peak height (FWHM) are typical. The resolution can be improved to 1.8% FWHM by modifying the DMA.

The measured mobility distribution of a polymer is correlated to its respective polymer mass in the following manner. Electrosprays of polymers including polyethyleneglycol (PEG) produce spherical polymer particles having bulk densities corresponding to a glassy or crystalline state, independent of whether they consist of single or multiple polymer chains. Since the polymer particles are spherical and much smaller than the gas mean free path, the mobility Z and mass m can be correlated to each other because both m and Z are related to particle diameter d according to H. Tammet in the Journal of Aerosol Science, Vol. 26, pp 459-475 (1995) and de la Mora *et al.* Trends in Analytical Chemistry, Vol 17, pp 328-339 (1998):

$$d = (6m/\pi\rho)^{1/3} \tag{1}$$

$$Z = [3q/2p \, (d + d_g)^2][(kT/2\pi m_g)^{1/2}/(1 + \pi\alpha/8)] \tag{2}$$

where p is the density of the spherical particle, q is the electrical charge, p is the pressure, T is temperature, mg is the molecular mass of the suspending gas, k is Boltzmann's constant, $d_g$ is the effective diameter for gas particle collisions and $\alpha$ is the corresponding accommodation coefficient. The $\alpha$ term is zero for elastic collisions and unity for completely inelastic collisions. A variety of measurements for small particles in air are consistent with equation (2) with $\alpha = 0.91$ as reported by S. Friedlander in Dust, Smoke and Haze, J Wiley and Sons, 2nd Ed., 2000 with $d_g$ reported as 0.53 nm. It follows from equations (1) and (2) that the quantities $m^{1/3}$ and $Z^{-1/2}$ are both linear in d and yield a linear relationship when plotted against each other:

$$Z^{-1/2} = A + B \, m^{1/3} \tag{3}$$

where the constants A and B are obtained by calibration as well as estimated from the information given above. A common procedure with larger polymer particles (d > 10 nm) is to invert the relation Z(d) to express the measured mobility in terms of a mobility diameter d(Z). This is done algebraically to include the mega Dalton range so that a plot of d(Z) versus $m^{1/3}$ is linear within the whole range of masses of interest to industrial polymers.

The mobility Z and inverse mobility 1/Z can be determined from the Millikan relation, where the Knudsen number Kn is the ratio between twice the mean free path of the carrier gas and the particle diameter d:

$$1/Z = 3\pi\mu \, (d + d_g)/ qC(Kn) \tag{4a}$$

$$C(Kn) = 1 + kn[1.257 + 0.4 \exp(-1.1/Kn)] \tag{4b}$$

$$Kn = [2\mu/(d + d_g)\rho_g][\pi m_g /2kT]^{1/2} \tag{4c}$$

where $\mu$ is the velocity coefficient of the gas and $\rho_g$ is the gas density. This yields d as a function of Z, which then allows d to be used in place of $Z^{-1/2}$, to yield a linear relationship between d and $m^{1/3}$, as shown in equation (5).

$$d = d_g + B_M m^{1/3} \tag{5}$$

Accordingly, the invention provides a method for determining molecular weight distribution of a polymer comprising the steps of

(a) electrospraying the polymer and one or more reference polymers;
(b) measuring ion mobility distributions for the polymer and one or more reference polymers using a high resolution differential mobility analyzer; and
(c) obtaining the molecular weight of the polymer as compared to the one or more reference polymers by calculating polymer mass from its corresponding measured polymer ion mobility.

The effectiveness of charge reduced electrospray mobility analysis for high resolution mass (molecular weight) distribution determination of water soluble polymers and water insoluble polymers has now been demonstrated according to the present invention. A representative example of a water soluble polymer is PEG. In one example, ion mobility spectra were measured for 12.6 kD PEG sample from electrosprays at dilute polymer concentration containing only one PEG molecule (chain) in a given droplet and increased polymer concentration where clusters of PEG molecules were present in a given droplet. At low polymer concentration, a mobility peak corresponding to a single chain PEG molecule (a unimer, n = 1) was observed. The gradual appearance of cluster ions where mobility peaks from unimer up to decamers (n = 10) are observed as the polymer concentration is increased from $1 \times 10^{-6}$ to $1 \times 10^{-6}$ M. From this example, the polymer mass range being probed is extended by a factor of 10, equivalent to a single 126 kD PEG chain, using a single polymer sample and cluster formation. Moreover, electrospray mobility analysis of a 120kD PEG sample can be extended to 720kD from mobility peaks corresponding to unimer up to hexamers using the method of the invention. One of the assumptions made in the method, especially in the case of clusters) is that the dry polymer particles are spherical and their density is independent of diameter, i.e. molecular weight. The validity of the assumption was verified using the two sets of experimental results for the PEG polymers, in that linear relationship was obtained between the inverse square root of polymer ion mobility versus the cube root of polymer mass (molecular weight). One advantage of the method is that the series of masses are internally dependent, consistent and is linear, as described above. Using the relation of mobility versus MW, it is calculated that a polydispersity of 1.001 gives a ion mobility peak with 5% FWHM. Thus the resolution of a conventional DMA imparts little to no observable peak broadening when measuring the molecular weight distribution of a polydisperse polymer, including synthetic, semi-synthetic and naturally occurring polymers. The two examples show that electrospray mobility analysis cluster data for the polymers provides the ability to accurately determine with high resolution molecular weight distribution of a polymer over a two orders of magnitude range, with resolution much better than GPC and a range beyond typically that seen in MALDI-MS spectra.

Electrospray mobility analysis of water insoluble polymers is performed in a similar manner using the method of the invention. A representative water insoluble polymer is polystyrene (PS). In one example, ion mobility spectra were measured for a 170kD PS sample from electrosprays at dilute polymer concentration containing only one PS molecule (chain) in a given droplet and increased polymer concentration where clusters of PS molecules were present in a given droplet. A unimer ( n= 1) ion mobility peak was observed at $2 \times 10^{-6}$ M. The gradual appearance of cluster ions where mobility peaks from unimer up to trimers (n = 3) are observed as the polymer concentration is increased to $1.7 \times 10^{-5}$ M, extending the molecular weight range up to 510 kD.

Any polymer whose molecular weight is unknown is determined using electrospray mobility analysis using one or more reference polymers. A reference polymer is a polymer whose molecular has been accurately determined using any conventional technique, such as GPC or MS. PEG standards are useful as reference polymer for water soluble polymers using the method of the invention. PS standards are useful as reference polymers for water insoluble polymers using the method of the invention. Any suitable water soluble polymer or water insoluble polymer can be employed as a reference, provided its molecular weight has been accurately determined. One advantage of clusters in electrospray mobility analysis is that the clusters ions also provide a series internal references. A mobility distribution for a polymer whose molecular weight is unknown is determined using the method of the invention relative to ion mobility distributions of one or more reference polymers.

A method for calibrating mobility distribution of a polymer to polymer mass comprising the steps of:

(a) obtaining one or more reference polymers having different molecular masses;
(b) measuring ion mobility for each reference polymer using a differential mobility analyzer;
(c) calculating particle diameters of the reference polymer ions from the measured mobility distribution; and
(d) obtaining a plot of polymer particle diameter d(Z) or polymer particle mobility ($Z^{-1/2}$) versus polymer mass, $(m)^{1/3}$ over a defined mass range.

The invention also provides a method for calibrating ion mobility of a polymer with its corresponding polymer mass and extending polymer mass range comprising the steps of

(a) obtaining a reference polymer having a narrow molecular weight distribution;
(b) generating a series of n cluster ions from the reference polymer and measuring ion mobility for each cluster ion using a differential mobility analyzer;
(c) calculating particle diameters of the reference polymer and corresponding cluster ions from the measured mobility distribution; and
(d) obtaining a plot of polymer particle diameter d(Z) or polymer particle mobility ($Z^{-1/2}$) versus polymer mass, $(m)^{1/3}$ over an extended mass range, n(m). One advantage of this method is that the series of reference masses are internally dependent, consistent and is linear, as described above.

As described above, mass analysis of both water soluble polymers and water insoluble polymers are performed using the method of invention. Water soluble polymers are electrosprayed from a buffer solution comprising water and a suitable salt to produce the required droplets. Suitable examples of water soluble polymers include, but are not limited to, for example polyalkylene oxides such as polyethylene glycol (PEG), polyproplyeneglycol (PPG), polyacrylic acid and its salts, polymethacrylic acid and its salts, polyitaconic acid and its salts, polycrotonic acid and its salts, polymaleic acid and its salts, styrenesulfonic acid and its salts, derivatives of styrenesulfonic acid and its salts, polyamines and its ammonium salts, polyaminoacrylates and its ammonium salts. Other suitable water soluble polymers include all polyelectrolytes such as poly(meth)acrylic acid copolymers and its salts, maleic acid anhydride copolymers, polysaccharides and its salts, polysaccharide derivatives and its salts, polyethylene imine and its salts, polyamidamines and its salts, ionenes and their salts, homo- and copolymers of cationic acrylic acid esters, gelatins and nucleic acids. It is contemplated that molecular weight distributions of all water soluble polymers can be determined using the method of the invention.

Mass analysis of water insoluble polymers are determined from electrosprays of the polymers in solvents having high dielectric constants. The term "dielectric constant" refers to the polarity of a liquid medium, including solvents and is defined by $\varepsilon$ in the equation $F = QQ'/\varepsilon r^2$, where F is the force of attraction between two charges Q and Q' separated by a distance r in the medium. The high dielectric solvents function to dissolve/disperse the polymer, have the required electrical conductivity an are free of impurities to yield uncontaminated polymer ions for mass analysis using the method of the invention. It is contemplated that molecular weight distributions of all water insoluble polymers can be determined using the method of the invention, provided they can be dissolved, suspended or dispersed in one or more suitable solvents having a sufficiently high dielectric constant for ionization.

Suitable examples of solvents having a high dielectric constant include, but are not limited to, solvents having a dielectric constant $\varepsilon > 2.0$. Suitable examples include for example dimethylsulfoxide (DMSO), acetonitrile, N-methylformamide, N,N-dimethylformamide (DMF), formamide, nitromethane, nitroethane, nitrobenzene, methanol, ethanol, propanol, isopropanol, 1-butanol, acetamide, ethylene glycol, 1,2-propanediol, 1,3-propanediol, allyl alcohol, hexamethylphosphoramide (HMPA), N-methyl-2-pyrrolidinone (NMP), 5-methyl-2-pyrrolidinone, 2-methyl-1-butanol, acetic anhydride, amyl alcohol, benzyl alcohol, cyclohexanone, glycolic nitrile, hydrogen cyanide (supercritical or in condensed phase at low temperatures), sulfur dioxide (supercritical or in condensed phase at low temperatures), hydrocyanic acid, isobutyronitrile, isobutyl alcohol, methylethylketone, methylpropylketone, methylcyclohexanone, N-methyl pyridine and tributyl phosphate.

Mass (molecular weight) analysis of a wide range of industrial polymers that are water insoluble are determined using the method of the invention. Suitable polymers include, but are not limited to, for example vinyl polymers such as polystyrene, polystyrene copolymers polyvinylacetate, polyvinylpyridines, polyvinylamines, polyvinylamides, polyvinyl ethers, condensation polymers such as polyesters and polyurethanes, polyethylenically unsaturated polymers such as polyethylene, polypropylene, poly(meth)acrylates, poly(meth)acrylate copolymers, polyalkyl(meth)acylates, polyalkyl(meth)acrylate copolymers, polyhydroxyakyl(meth)acrylates, polyacrylonitrile, polyacrylonitrile copolymers, polyacrylamide, poly(meth)acrylamide and poly(meth)acrylamide copolymers, polyurethanes and polyesters. Other suitable examples of water insoluble polymers include cross-linked polymers of the polymers listed.

Water insoluble acrylic polymers useful in the invention are prepared by conventional polymerization techniques including solution, suspension and emulsion polymerization. For example, dispersions of the latex polymer particles are prepared according to processes including those disclosed in U.S. Patent Nos. 4,427,836; 4,469,825; 4,594,363; 4,677,003; 4,920,160; and 4,970,241. The latex polymer particles may also be prepared, for example, by polymerization techniques disclosed in European Patent Applications EP 0 267 726; EP 0 331 421; EP 0 915 108 and U.S. Patent Nos. 4,910,229; 5,157,084; 5,663,213 and 6,384,104.

As used herein, the term "(meth)acrylic" refers to either the corresponding acrylic or methacrylic acid and derivatives; similarly, the term "alkyl (meth)acrylate" refers to either the corresponding acrylate or methacrylate ester.

Mass analysis of polymers via mobility measurements provides several important advantages. It allows the mass (molecular weight) analysis of polymers that cannot be determined using GPC or directly determined using conventional MS instruments. The mass analysis is performed on a fast time scale. The mobility of polymer ions can be correlated from first principles to polymer particle size and shape.

Polymers analyzed in accordance with the invention include both water soluble and water insoluble polymers having weight average molecular weights between 1 kilodaltons (kD) and 500,000 kD.

The method is also generally applicable for the determination of molecular weight of many types of industrial polymers. According to one embodiment of the invention, the mobility versus mass relation is determined for each polymer analyzed. According to separate embodiment the mobility versus mass relation is determined by transforming the relation obtained with one polymer for use with another polymer. The experimental data indicate the density of the single chain particle is similar to that of the bulk polymer and that bulk densities are either measured or estimated.

Some embodiments of the invention are described in detail in the following Examples.

EXAMPLE 1

MASS ANALYSIS OF A WATER SOLUBLE POLYMER

Aqueous solutions of polyethylene glycol (PEG) in a 10millimolar (mM) ammonium acetate buffer were electrosprayed. The buffer included a 50/50 (v/v) water methanol solution with 10mM ammonium acetate. Carbon dioxide was used as a gas for electrospraying. Commercially available PEG samples were obtained (Polymer Laboratories, Amherst MA 01003). The molecular weight were determined independently by the manufacturer using GPC and light scattering measurements and are listed in Table 1.

Table 1.

| PEG samples. | | |
|---|---|---|
| PEG sample Mw (g/mol) | Polydispersity ratio Mw/Mn | Concentration moles/Liter |
| 4,120 | 1.02 | $5*10^{-3}$ |
| 9,000 | 1.01 | $5*10^{-4}$ |
| 12,600 | 1.01 | $5*10^{-4}$ |
| 22,800 | 1.02 | $2*10^{-4}$ |
| 50,100 | 1.02 | $2*10^{-5}$ |
| 120,000 | 1.02 | $7*10^{-5}$ |

The mobility distribution of the polymer ions formed was measured in air using a high resolution DMA and compared with corresponding matrix assisted laser desorption ionization (MALDI) mass spectrometry (MS) data. Mobility spectra were obtained for each PEG sample. Results confirmed that any peak broadening introduced using the method of the invention does not significantly affect the calculated MWD for the narrowest available PEG sample. The relationship between the mobility of the polymer ions and its mass were determined relative to PEG mass standards having narrow MWD. PEG samples were electrosprayed at concentrations (Table 1) low enough to yield no more than one polymer chain per droplet. To maximize the information obtainable from the PEG standards, more concentrated solutions were also electrosprayed and analyzed by DMA. The gradual appearance of cluster ions including one PEG molecule up to 6 molecules (hexamer) were observed as the concentration increased from $1*10^{-6}$ to $1*10^{-4}$ moles/Liter (M). The clustering process has the advantage of magnifying the effective number of mass standards available by a factor of n(m).

A reliable relation between polymer ion mobility and polymer mass was obtained using the commercially available PEG standards. The results confirmed the PEG particles are spherical with bulk densities corresponding to a glassy or crystalline state, independently of whether they consist of single or multiple polymer chains. A constant shape and density for the particles formed is useful for accurately determining the mass from the measured mobility, since Z is a function of cross-sectional area, which depends on shape and volume which in turn depends on density.

Reported values for the bulk density of PEG are 1.204 $g/cm^3$ at Mw = 3,400 g/mol and 1.21 $g/cm^3$ at Mw = 6,000 g/mol at 298K. Using equations (1)- (5) a linear plot of d(Z) versus $m^{1/3}$ was obtained for the PEG standards. A fit of the data using equation (5) yields $d_g$ = 0.453 nm and $B_M$ = 0.1364 $nm/(g/mol)^{1/3}$. From the slope of the line $B_M$, a particle density of 1.25 $g/cm^3$ is obtained consistent with the bulk density of PEG.

EXAMPLE 2

MASS ANALYSIS OF A WATER INSOLUBLE POLYMER

Polystyrene polymers having narrow mass distributions and mean molecular weights in the range 9kD < Mn < 170kD are electrosprayed from their solutions in NMP seeded with 5% by volume of trifluoroacetic acid. The polystyrene mass standards were obtained commercially and summarized in Table 2.

Table 2.

| Polystyrene samples. | | |
|---|---|---|
| Psty sample Mw (g/mol) | Polydispersity ratio Mw/Mn | Concentration moles/Liter |
| 9,200 | 1.03 | $2.1*10^{-3}$ |
| 34,500 | 1.04 | $4.0*10^{-4}$ |
| 68,000 | 1.04 | $2.1*10^{-4}$ |
| 170,000 | 1.03 | $7.9*10^{-5}$ |

The mobility distribution of the polymer ions formed was measured in air using a high resolution DMA. Mobility spectra were obtained for each polystyrene sample. The polystyrene mass standards used at concentrations approaching $1*10^{-4}$ moles/Liter produce several well-defined mobility peaks associated to the formation of particles containing from one up to 6 (hexamer) polystyrene molecules.
A reliable relation between polymer ion mobility and polymer mass was obtained using the commercially available polystyrene standards. The results confirmed the polystyrene particles are spherical with bulk densities corresponding to a glassy or crystalline state, independently of whether they consist of single or multiple polymer chains. The use of 4 polystyrene mass standards (Mn (kD) 9.2, 45, 68 and 170) yields 15 mass versus mobility data that establish a linear relationship for $(Z^{-1/2})$ versus $m^{1/3}$ from 9kD to 170 kD. Spherical polymer particles were confirmed from electrospraying and mobility analysis consistent with the measured bulk density of polystyrene. Using equations (1)- (5) a linear plot of $(Z^{-1/2})$ versus $m^{1/3}$ was obtained for the PEG standards. A fit of the data using equation (5) and from the slope of the line $B_M$, a particle density of 1.067 g/cm$^3$ is obtained, consistent with the bulk density of PS. The bulk density of PS ranges from 1.040 to 1.080 g/cm$^3$. The highest molecular weight PS sample was consistent within 6%.

**Claims**

1. An apparatus for the direct mass analysis of molecules and macromolecules comprising: an electrospray generator for producing electrosprays of an analyte comprising molecules and macromolecules, the electrospray generator including an ionization source for charge reduction of gas phase analyte ions coupled with a time of flight mass spectrometer for direct mass analysis of the analyte.

2. The apparatus according to claim 1, wherein the molecular weight of the analyte is between 1 kilodalton (kD) and 500,000 kD.

3. The apparatus according to claim 1, wherein the analyte is water soluble or water insoluble polymer.

4. The apparatus according to claim 3, wherein water insoluble polymer is electrosprayed using a solvent or liquid carrier having a dielectric constant of at least 2.0.

5. The apparatus according to claim 4, wherein the solvent or liquid carrier is selected is from the group consisting of dimethylsulfoxide (DMSO), acetonitrile, N-methylformamide, N,N-dimethylformamide (DMF), formamide, nitromethane, nitroethane, nitrobenzene, methanol, ethanol, propanol, 1-butanol, acetamide, ethylene glycol, 1,2-propanediol, 1,3-propanediol, allyl alcohol, hexamethylphosphoramide (HMPA), N-methyl-2-pyrrolidinone (NMP), 5-methyl-2-pyrrolidinone, 2-methyl-1-butanol, acetic anhydride, amyl alcohol, benzyl alcohol, cyclohexanone, glycolic nitrile, hydrogen cyanide, hydrocyanic acid, isobutyronitrile, isobutyl alcohol, methylethylketone, methylpropylketone, methylcyclohexanone, N-methyl pyridine and tributyl phosphate.

6. A method for directly determining the molecular weight of molecules and macromolecules comprising the steps of

(a) generating an electrospray of an analyte comprising molecules and macromolecules; and
(b) measuring the mass ratio of the analyte by directing the charge reduced electrospray of the analyte into a time of flight mass spectrometer.

7. The method according to claim 6, wherein the molecular weight of the analyte is between 1 kilodalton (kD) and 500,000 kD.

8. The method according to claim 6, wherein the analyte is water soluble or water insoluble polymer.

9. The method according to claim 8, wherein the water insoluble polymer is electrosprayed using a solvent or liquid carrier having a dielectric constant of at least 2.0.

10. The method according to claim 9, wherein the solvent or liquid carrier is selected is from the group consisting of dimethylsulfoxide (DMSO), acetonitrile, N-methylformamide, N,N-dimethylformamide (DMF), formamide, nitromethane, nitroethane, nitrobenzene, methanol, ethanol, propanol, 1-butanol, acetamide, ethylene glycol, 1,2-propanediol, 1,3-propanediol, allyl alcohol, hexamethylphosphoramide (HMPA), N-methyl-2-pyrrolidinone (NMP), 5-methyl-2-pyrrolidinone, 2-methyl-1-butanol, acetic anhydride, amyl alcohol, benzyl alcohol, cyclohexanone, glycolic nitrile, hydrogen cyanide, hydrocyanic acid, isobutyronitrile, isobutyl alcohol, methylethylketone, methylpropylketone, methylcyclohexanone, N-methyl pyridine and tributyl phosphate.